# EUROPEAN PATENT APPLICATION

(11) **EP 3 354 274 A1**
(43) Date of publication of application: **01.08.2018**
(21) Application number: 17152992.8
(22) Date of filing: 25.01.2017
(51) Int. Cl.: A61K 38/08, A61K 31/40, A61K 31/427, A61K 31/496, A61K 31/7036, A61P 31/04

(54) **THERAPEUTIC COMBINATIONS FOR THE TREATMENT OF BACTERIAL INFECTIONS**

(71) Applicant: SETLANCE S.r.l., 53100 Siena (IT)
(72) Inventor: FALCIANI, Chiara, 53100 SIENA (IT); PINI, Alessandro, 53100 SIENA (IT); BRACCI, Luisa, 53100 SIENA (IT); BRUNETTI, Jlenia, 53100 SIENA (IT); ROSSOLINI, GianMaria, 50134 FIRENZE (IT); POLLINI, Simona, 50134 FIRENZE (IT)
(74) Representative: Bianchetti Bracco Minoja S.r.l.

(57) **Abstract**

There are disclosed therapeutic combinations of an antibacterial peptide and an antibiotic drug for use in the treatment of infections caused by Gram-negative pathogens, including bacterial strains resistant to common antibiotics. Also disclosed are pharmaceutical preparations and compositions containing the antibacterial peptide and antibiotics.

SEQUENCE LISTING

<110>  SETLANCE S.R.L.

<120>  THERAPEUTIC COMBINATIONS FOR THE TREATMENT OF BACTERIAL
       INFECTIONS

<130>  3242EUR

<160>  1

<170>  PatentIn version 3.5

<210>  1
<211>  9
<212>  PRT
<213>  Artificial Sequence

<220>
<223>  synthetic peptide

<400>  1

Lys Lys Ile Arg Val Arg Leu Ser Ala
1               5

## Description

The present invention provides a therapeutic combination of an antibacterial peptide and an antibiotic drug for use in the treatment of infections caused by Gram-negative pathogens. The therapeutic combination showed a synergistic activity against bacterial strains resistant to common antibiotics. The invention further provides pharmaceutical preparations and compositions containing the antibacterial peptide and antibiotics in admixture with suitable excipients.

### Background to the invention

The global threat of a new "pre-antibiotic" era has now been acknowledged by all sectors of the Society, not only the scientific community, but also public health institutions and governments. Due to the spread of multi-drug resistant (MDR) and extremely drug-resistant (XDR) bacterial pathogens, even common infections may transform into conditions that are untreatable with the standard of care antibiotics [1-2].

Great efforts are now directed to face this urgent medical need. New antibiotics are required, together with new combination treatments, to control infections caused by MDR and XDR pathogens [3].

SET-M33 is an antimicrobial peptide (AMP) that has been extensively studied in recent years [4-7]. It is active in vitro and in vivo against Gram-negative bacteria, while lacking immunogenicity [8] and hemolytic activity, and showing an acceptable toxicity profile with human cells and in mice [9-10]. This AMP is currently being developed as a new treatment option against MDR and XDR Gram-negative infections. In fact, SET-M33 already showed efficacy against a number of Gram-negative MDR and XDR clinical isolates, including isolates from Cystic Fibrosis patients, and exhibited ability also in eradicating biofilms [6].

The combination of anti-infective compounds to expand spectrum, improve the therapeutic outcome and even diminish the emergence of resistance is a well-established strategy in antimicrobial chemotherapy [11-13].

Synergistic activity of AMPs, particularly of colistin, in combination with different antimicrobial agents has been investigated in previous studies. Colistin has frequently been shown synergy with rifampin and carbapenems against Gram negatives, including carbapenem-resistant *K. pneumoniae* and Gram-negative non-fermenters [21-23], and to a lesser extent with aztreonam, fluoroquinolones and aminoglycosides [21, 24-26]. Other AMPs were also reported as synergistic in combination mostly with rifampin and β-lactams [20, 27-28], whereas data on synergy with other agents remain more limited or controversial [29-31].

### Known art

WO2006/006195 discloses antibacterial peptides, including the peptide QKKIRVRLSA (M6), exhibiting antimicrobial activity against several bacterial species, with reduced cytotoxicity and low haemolysis rate. The peptides are in linear form or multimerised on a skeleton of polyacrylamide, dextrane or ethylene glycol units and preferably the peptides are provided in the form of Multiple Antigenic Peptides.

EP2344178 discloses antibacterial peptide sequences, including the sequence KKIRVRLSA, which corresponds to the M6 peptide disclosed in WO2006/006125 deprived of the first amino acid Gln. The peptides produce better antimicrobial activity, besides improved stability and batch to batch homogeneity, and are provided in monomeric or dendrimeric structure and particularly in the Multiple Antigen Peptide form.

WO2010/038220 discloses antimicrobial peptides, including the KKIRVRLSA peptide, which are in monomeric or dendrimeric structure, preferably in the Multiple Antigen Peptide form.

EP2595496 discloses the peptide KKIRVRLSA characterized in that all amino acids are in D-configuration (D-M33), either in linear form or multimerized on a skeleton of polyacrylamide, dextran or glycol units.

### Description of the invention

In the present invention, the peptide SET-M33 - also referred to as "M33" and having the sequence KKIRVRLSA according to one letter amino acid code-has been assessed *in vitro* for antimicrobial activity against Gram-negative pathogens, in combination with standard of care antibiotics.

It was surprisingly found that this peptide produces synergistic effects in combination with rifampin, meropenem, aztreonam and tobramycin against multidrug- and extensively drug-resistant strains of the Gram-negative pathogens *Klebsiella pneumoniae, Pseudomonas aeruginosa* and *Acinetobacter baumannii.*

All the tested strains exhibited a resistant phenotype toward major classes of antibiotics, including quinolones, aminoglycosides and β-lactams. All the strains were carbapenemase producers, with the *K. pneumoniae, P. aeruginosa* and *A. baumannii* strains encoding a KPC-type enzyme, a metallo-β-lactamase (VIM-1 and IMP-13 enzymes) and an OXA-24 enzyme, respectively. All the strains belonged in successful high-risk clones that are known to play a major role in the spread of antibiotic resistance [17].

Notably, the tested combinations proved successful against colistin-resistant strains. This is even more surprising considering that a common mechanism of action for colistin and SET-M33 has previously been hypothesized [10].

Accordingly, it is an objective of the present invention to provide a therapeutic combination of the antibacterial peptide KKIRVRLSA with an antimicrobial agent selected from the group consisting of meropenem, rifampin, aztreonam and tobramycin, for use in the treatment of human or animal infections caused by one or more of the following bacterial species: *Klebsiella pneumoniae, Pseudomonas aeruginosa* and *Acinetobacter baumannii.*

The peptide KKIRVRLSA can be prepared according to known procedures, for example as described in Brunetti J et al. In vitro and in vivo efficacy, toxicity, bio-distribution and resistance selection of a novel antibacterial drug candidate. Sci Rep. 2016; 6:26077. doi: 10.1038/srep26077. It can be provided in linear form or in multimeric form, e.g. in dendrimeric form and particularly two-branched and four branched-form, or in Multiple Antigen Peptide form, as described in EP2344178 and in WO2010/038220. The amino acid residues contained in the KKIRVRLSA peptide can be in either L- or D-configuration; the peptide KKIRVRLSA wherein all the amino acids are in D-configuration is described in EP2595496.

In one preferred embodiment, the peptide M33 or an analogue or derivative thereof, is combined with an antimicrobial agent selected from meropenem and aztreonam and the combination is used in the treatment of infections caused by the bacterial species *Pseudomonas aeruginosa.*

In another preferred embodiment, the peptide M33 or an analogue or derivative thereof is combined with an antimicrobial agent selected from meropenem, aztreonam, tobramycin and rifampin and the combination is used in the treatment of infections caused by the bacterial species *Acinetobacter baumannii.*

In a further preferred embodiment, the peptide M33 or an analogue or derivative thereof is combined with the antimicrobial agent rifampin and the combination is used in the treatment of infections caused by the bacterial species *Klebsiella pneumoniae.*

In order to identify the combination of peptide M33 and antibiotic(s) which is most effective for the treatment of an infected individual, a biological sample from said individual can be assayed for responsiveness to different combinations of the invention. Accordingly, in a further embodiment the invention provides a combination as above defined, for use in a method of treating infections which comprises:
1) providing a sample of biologic fluid or tissue from a subject diagnosed positive for infection by one or more of the following bacterial species: *Klebsiella pneumoniae, Pseudomonas aeruginosa* and *Acinetobacter baumannii;*
2) testing in vitro the responsiveness of the infectious strain to a combination as defined above
   and, if the strain growth is inhibited in vitro by the combination,
3) administering said combination to the subject.

The invention further provides a kit for treating infections caused by one or more Gram-negative pathogens selected from *Klebsiella pneumoniae, Pseudomonas aeruginosa* and *Acinetobacter baumannii,* wherein the peptide M33 and one or more antibiotics selected from meropenem, rifampin, aztreonam, and tobramycin, are placed in separate containers together with pharmaceutically acceptable carriers and excipients and are provided in suitable forms for simultaneous or separate, e.g. sequential administration to a subject in need thereof.

The invention further provides a pharmaceutical composition containing the peptide SET-M33 and one or more antibiotic drugs selected from meropenem, rifampin, aztreonam and tobramycin, together with pharmaceutically acceptable excipients. The pharmaceutical composition is suitable for oral, topic or parenteral administration and it is preferably in the form of a tablet, capsule, injectable solution, eyewash, mouthwash, spray, aerosol, cream or ointment. The pharmaceutical composition of the invention may be formulated according to standard methods such as those described in Remington's Pharmaceuticals Sciences Handbook", Mack Pub. Co., NY, USA, XVII ed. Pharmaceutically acceptable excipients that may be used to formulate the composition of the invention are, in particular, described in the Handbook of Pharmaceuticals Excipients, American Pharmaceutical Association (Pharmaceutical Press; 6th Revised edition, 2009).

The kit and pharmaceutical composition according to the invention contain an effective amount of peptide and antibiotic drug. Suitable daily amounts of peptide M33 range from 0.5 mg/Kg to 50mg/Kg, preferably from 3 mg/Kg to 15 mg/Kg. The amount of antibiotic drug is generally within the intervals of the standard of care and will depend on the specific molecule used, on the severity of the disease and on the general conditions of the patient to be treated, or it can be assessed on the basis of the patient's responsiveness to the treatment.

The following experimental section further illustrates the invention.

### Experimental section

### Materials and Methods

### Peptide synthesis

SET-M33 was prepared by solid-phase synthesis through standard Fmoc chemistry, using a Syro multiple peptide synthesizer (MultiSynTech, Witten, Germany). Side chain protecting groups were 2,2,4,6,7-pentamethyldihydrobenzofuran-5-sulfonyl for R, t-butoxycarbonyl for K and t-butyl for S (Iris Biotech GmbH, Marktredwitz, Germany). The final product was cleaved from the solid support, deprotected by treatment with TFA containing triisopropylsilane and water (95/2.5/2.5), and precipitated with diethyl ether. Final peptide purity and identity was confirmed by reversed phase chromatography on a Phenomenex Jupiter C18 analytical column (300 Å, 250 x 4.6 mm) and by mass spectrometry.

### Bacterial strains

Activity of SET-M33 and antibiotics was evaluated on six well characterized clinical strains. The strains were selected according to their resistance phenotypes and genotypes. A detailed description of each strain is reported in Table.

### In vitro susceptibility testing

MICs were determined using a standard broth microdilution assay according to the guidelines of the Clinical and Laboratory Standards Institute [14].

Antimicrobial activity in combination was evaluated, in duplicate for each of the six strains, with the checkerboard method [15] using cation-adjusted Mueller-Hinton broth (MHB) in 96-well microtiter plates (Sarstedt, Inc., Newton, NC). Assays were performed using a final bacterial inoculum of 5x10⁴ CFU/well in a final volume of 100 µl. Results were recorded after 20 h of incubation at 35°C. The total fractional inhibitory concentration (∑FIC) for each antibiotic combination was calculated according to EUCAST definitive document E.Def 1.2 [16] as follows: ∑FIC equals FIC of agent A plus FIC of agent B, where the FIC of agent A or B is the MIC of agent A or B in the presence of the other divided by the MIC of agent A or B alone. Results were interpreted according to the following definition: a ∑IC of ≤0.5 indicated synergism, a ∑FIC of >0.5 to 1 indicated additivity, a ∑FIC of> 1 to <2 indicated indifference, and a ∑FIC of ≥2 antagonism.

### Results

The MIC values of SET-M33 varied between 4 and 32 µg/mL and the MICs of the other antibiotics were always in the resistant range according to the EUCAST clinical breakpoints [19], when available.

SET-M33 and meropenem were synergic with two of the six strains (one *P. aeruginosa* and one *A. baumannii*) and showed an additive effect with the remaining strains. A synergistic effect was observed for SET-M33 and rifampin combination with three out of six strains, including *K pneumoniae* and *A. baumannii strains.* SET-M33 and aztreonam showed synergy with one of the *P. aeruginosa* and both *A. baumannii* strains. Synergy was also observed with one of the *A. baumannii* strains when SET-M33 was used in combination with tobramycin. (Table). Additivity was only observed when SET-M33 was used in combination with ciprofloxacin (not shown). Notably, an antagonistic effect was never detected.

Analysis of the antibiotic combinations resulting in growth inhibition revealed that, in most cases, SET-M33 MIC values were moderately affected (when a synergistic effect was present, e. g. in combination with rifampin, aztreonam and tobramycin) or not affected (when the effect was additive or indifferent). On the other hand, the MIC values of conventional antibiotics were remarkably affected, with a decrease down to 128-fold in cases of synergistic effect (e. g. for meropenem and rifampin) (Table).

Interestingly, in combination with rifampin or aztreonam, SET-M33 showed synergistic activity even on colistin-resistant strains of *K. pneumoniae.*

**Table. MIC values and synergistic activities of different antimicrobial combinations against 6 MDR/XDR Gram-negative strains. MICs of each antimicrobial agents and MIC decrease when used in combination are shown; synergy is indicated by shading.**

| ATB in combination ^{a} | Strain | Strain features ^{b, c} | MIC (µg/mL) | | MIC in combination (µg/mL) | | ∑FIC |
|---|---|---|---|---|---|---|---|
| | | | SET-M33 | ATB | SET-M33 | ATB | |
| SET-M33 + meropenem | K. pneumoniae KKBO-4 | XDR, KPC, colR | 16 | 64 | 8 | 32 | 1.0 |
| | K. pneumoniae 044-R | XDR, KPC, colR | 16 | 64 | 8 | 32 | 1.0 |
| | P. aeruginosa 854 | XDR, VIM-1 | 16 | 512 | 8 | 16 | 0.5 |
| | P. aeruginosa AV65 | XDR, IMP-13 | 8 | 256 | 4 | 64 | 0.8 |
| | A. baumannii VA566/00 | MDR, OXA-24 | 8 | 512 | 4 | 4 | 0.5 |
| | A. baumannii N50 | MDR, OXA-24, colR | 8 | 256 | 4 | 64 | 0.8 |
| SET-M33 + rifampin | K. pneumoniae KKBO-4 | XDR, KPC, colR | 32 | 128 | 4 | 32 | 0.4 |
| | K. pneumoniae 044-R | XDR, KPC, colR | 16 | 64 | 4 | 16 | 0.5 |
| | P. aeruginosa 854 | XDR, VIM-1 | 16 | 32 | 8 | 8 | 0.8 |
| | P. aeruginosa AV65 | XDR, IMP-13 | 16 | 128 | 8 | 64 | 1.0 |
| | A. baumannii VA566/00 | MDR, OXA-24 | 16 | 256 | 4 | 2 | 0.3 |
| | A. baumannii N50 | MDR, OXA-24, colR | 16 | 64 | 8 | 4 | 0.6 |
| SET-M33 + aztreonam | K. pneumoniae KKBO-4 | XDR, KPC, colR | 16 | 2048 | 8 | 2048 | 1.5 |
| | K. pneumoniae 044-R | XDR, KPC, colR | 16 | 4096 | 8 | 2048 | 1.0 |
| | P. aeruginosa 854 | XDR, VIM-1 | 16 | 128 | 8 | 64 | 1.0 |
| | P. aeruginosa AV65 | XDR, IMP-13 | 16 | 32 | 8 | 1 | 0.5 |
| | A. baumannii VA566/00 | MDR, OXA-24 | 16 | 512 | 4 | 16 | 0.3 |
| | A. baumannii N50 | MDR, OXA-24, colR | 16 | 128 | 4 | 32 | 0.5 |

| ATB in combination ^{a} | Strain | Strain features ^{b,c} | MIC (µg/mL) | | MIC in combination (µg/mL) | | ∑FIC |
|---|---|---|---|---|---|---|---|
| | | | SET-M33 | ATB | SET-M33 | ATB | |
| SET-M33 + tobramycin | K. pneumoniae KKBO-4 | XDR, KPC, colR | 32 | 32 | 16 | 16 | 1.0 |
| | K. pneumoniae 044-R | XDR, KPC, colR | 32 | 32 | 16 | 16 | 1.0 |
| | P. aeruginosa 854 | XDR, VIM-1 | 16 | 256 | 8 | 256 | 1.5 |
| | P. aeruginosa AV65 | XDR, IMP-13 | 4 | 128 | 2 | 128 | 1.5 |
| | A. baumannii VA566/00 | MDR, OXA-24 | 32 | 16 | 8 | 4 | 0.5 |
| | A. baumannii N50 | MDR, OXA-24, colR | 16 | 8 | 8 | 0.5 | 0.6 |

| | | | | | | | |
|---|---|---|---|---|---|---|---|
| ^{a} ATB, antimicrobial agent. ^{b} colR, colistin resistant; KPC, *Klebsiella pneumoniae* carbapenemase; VIM-1, Verona integron-borne metallo-β-lactamase, allelic variant 1; IMP-13, IMP-type imipenemase, allelic variant 13; OXA-24, oxacillinase, allelic variant 24. | | | | | | | |

### REFERENCES

[1] Rossolini GM, Arena F, Pecile P, Pollini S. Update on the antibiotic resistance crisis. Curr Opin Pharmacol. 2014;18:56-60. doi: 10.1016/j.coph.2014.09.006.
[2] Viale P, Giannella M, Tedeschi S, Lewis R. Treatment of MDR-Gram negative infections in the 21 st century: a never ending threat for clinicians. Curr Opin Pharmacol. 2015; 24:30-7. doi: 10.1016/j.coph.2015.07.001.
[3] Bassetti M, Righi E. New antibiotics and antimicrobial combination therapy for the treatment of gram-negative bacterial infections. Curr Opin Crit Care. 2015; 21(5):402-11. doi: 10.1097/MCC.0000000000000235.
[4] Pini A, Giuliani A, Falciani C, Runci Y, Ricci C, Lelli B, Malossi M, Neri P, Rossolini GM, Bracci L. Antimicrobial activity of novel dendrimeric peptides obtained by phage display selection and rational modification. Antimicrob Agents Chemother 2005, 49: 2665-72.
[5] Pini A, Falciani C, Mantengoli E, Bindi S, Brunetti J, Iozzi S, Rossolini GM, Bracci L. A novel tetrabranched antimicrobial peptide that neutralizes bacterial lipopolysaccharide and prevents septic shock in vivo. FASEB J 2010, 24: 1015-22.
[6] Pini A, Lozzi L, Bernini A, Brunetti J, Falciani C, Scali S, Bindi S, Di Maggio T, Rossolini GM, Niccolai N, Bracci L. Efficacy and toxicity of the antimicrobial peptide M33 produced with different counter-ions. Amino Acids 2012, 43: 467-73.
[7] Falciani C, Lozzi L, Scali S, Brunetti J, Bracci L, Pini A. Site-specific pegylation of an antimicrobial peptide increases resistance to Pseudomonas aeruginosa elastase. Amino Acids 2014, 46: 1403-7.
[8] Pini A, Giuliani A, Falciani C, Fabbrini M, Pileri S, Lelli B, Bracci L. Characterization of the branched antimicrobial peptide M6 by analyzing its mechanism of action and in vivo toxicity. J Pept Sci 2007, 13: 393-9
[9] Falciani C, Lozzi L, Pollini S, Luca V, Carnicelli V, Brunetti J, Lelli B, Bindi S, Scali S, Di Giulio A, Rossolini GM, Mangoni ML, Bracci L, Pini A. Isomerization of an antimicrobial peptide broadens antimicrobial spectrum to Gram-positive bacterial pathogens. PLoS One 2012, 7: e46259.
[10] Brunetti J, Falciani C, Roscia G, Pollini S, Bindi S, Scali S, Arrieta UC, Gómez-Vallejo V, Quercini L, Ibba E, Prato M, Rossolini GM, Llop J, Bracci L, Pini A. In vitro and in vivo efficacy, toxicity, bio-distribution and resistance selection of a novel antibacterial drug candidate. Scientific Reports 2016, 6: 26077.
[11] Kalan L, Wright GD. 2011. Antibiotic adjuvants: multicomponent antiinfective strategies. Expert Rev Mol Med 13: e5. doi: 10.1017/S1462399410001766.
[12] Hu Y, Liu A, Vaudrey J, Vaiciunaite B, Moigboi C, McTavish SM, Kearns A, Coates A. 2015. Combinations of β-lactam or aminoglycoside antibiotics with plectasin are synergistic against methicillin-sensitive and methicillin-resistant Staphylococcus aureus. PLoS One 10: e0117664. http://dx.doi.org/10.1371/journal.pone.0117664
[13] Soothill G, Hu Y, Coates A. Can we prevent antimicrobial resistance by using antimicrobials better? Pathogens 2013; 2: 422-435.
[14] CLSI Methods for dilution antimicrobial susceptibility tests for bacteria that grow aerobically; Approved Standard-Tenth Edition. CLSI document M07-A10. Wayne, PA (2015).
[15] Marques MB, Brookings ES, Moser SA, Sonke PB, Waites KB. Comparative in vitro antimicrobial susceptibilities of nosocomial isolates of Acinetobacter baumannii and synergistic activities of nine antimicrobial combinations. Antimicrob. Agents Chemother. 1997; 41:881-885.
[16] European Committee for Antimicrobial Susceptibility Testing. 2000. Terminology relating to methods for the determination of susceptibility of bacteria to antimicrobial agents. EUCAST definitive document E.Def 1.2., 2000, EUCAST, Basel, Switzerland.
[17] Woodford N, Turton JF, Livermore DM. Multiresistant Gram-negative bacteria: the role of high-risk clones in the dissemination of antibiotic resistance. FEMS Microbiol Rev. 2011; 35(5): 736-55.
[18] Fiel SB. Aerosolized antibiotics in cystic fibrosis: an update. Expert Rev Respir Med. 2014; 8(3):305-14. doi: 10.1586/17476348.2014.896205.
[19] EUCAST clinical breakpoint v. 6.0, www.eucast.org/clinical breakpoints, accessed November 2016.
[20] Soren O, Brinch KS, Patel D, Liu Y, Liu A, Coates A, Hu Y. Antimicrobial peptide novicidin synergizes with rifampin, ceftriaxone, and ceftazidime against antibiotic-resistant enterobacteriaceae in vitro. Antimicrob Agents Chemother. 2015; 59(10):6233-40. doi: 10.1128/AAC.01245-15.
[21] Tascini C, Tagliaferri E, Giani T, Leonildi A, Flammini S, Casini B, Lewis R, Ferranti S, Rossolini GM, Menichetti F. Synergistic activity of colistin plus rifampin against colistin-resistant KPC-producing Klebsiella pneumoniae. Antimicrob Agents Chemother. 2013; 57(8): 3990-3. doi: 10.1128/AAC.00179-13.
[22] Zusman O, Avni T, Leibovici L, Adler A, Friberg L, Stergiopoulou T, Carmeli Y, Paul M. Systematic review and meta-analysis of in vitro synergy of polymyxins and carbapenems. Antimicrob Agents Chemother. 2013; 57(10):5104-11. doi: 10.1128/AAC.01230-13.
[23] Nastro M, Rodriguez CH, Monge R, Zintgraff J, Neira L, Rebollo M, Vay C, Famiglietti A. Activity of the colistin-rifampicin combination against colistin-resistant, carbapenemase-producing Gram-negative bacteria. J Chemother. 2014; 26(4):211-6. doi: 10.1179/1973947813Y.0000000136.
[24] Bae S, Kim MC, Park SJ, Kim HS, Sung H, Kim MN, Kim SH, Lee SO, Choi SH, Woo JH, Kim YS, Chong YP. In vitro synergistic activity of antimicrobial agents in combination against clinical isolates of colistin-resistant Acinetobacter baumannii. Antimicrob Agents Chemother. 2016 Sep 6. pii: AAC.00839-16.
[25] Safarika A, Galani I, Pistiki A, Giamarellos-Bourboulis EJ. Time-kill effect of levofloxacin on multidrug-resistant Pseudomonas aeruginosa and Acinetobacter baumannii: synergism with imipenem and colistin. Eur J Clin Microbiol Infect Dis. 2015; 34(2):317-23. doi: 10.1007/s10096-014-2231-7.
[26] Tängdén T, Hickman RA, Forsberg P, Lagerbäck P, Giske CG, Cars O. Evaluation of double- and triple-antibiotic combinations for VIM- and NDM-producing Klebsiella pneumoniae by in vitro time-kill experiments. Antimicrob Agents Chemother. 2014; 58(3):1757-62. doi: 10.1128/AAC.00741-13.
[27] Vaara M, Siikanen O, Apajalahti J, Frimodt-Moller N, Vaara T. Susceptibility of carbapenemase-producing strains of Klebsiella pneumoniae and Escherichia coli to the direct antibacterial activity of NAB739 and to the synergistic activity of NAB7061 with rifampicin and clarithromycin. J Antimicrob Chemother. 2010; 65(5):942-5. doi: 10.1093/jac/dkq040.
[28] Zhou Y, Peng Y. Synergistic effect of clinically used antibiotics and peptide antibiotics against Gram-positive and Gram-negative bacteria. Exp Ther Med. 2013; 6(4):1000-1004.
[29] Bozkurt-Guzel C, Savage PB, Gerceker AA. In vitro activities of the novel ceragenin CSA-13, alone or in combination with colistin, tobramycin, and ciprofloxacin, against Pseudomonas aeruginosa strains isolated from cystic fibrosis patients. Chemotherapy. 2011; 57(6):505-10. doi: 10.1159/000335588.
[30] Zhang Y, Liu Y, Sun Y, Liu Q, Wang X, Li Z, Hao J. In vitro synergistic activities of antimicrobial peptide brevinin-2CE with five kinds of antibiotics against multidrug-resistant clinical isolates. Curr Microbiol. 2014; 68(6):685-92. doi: 10.1007/s00284-014-0529-4.
[31] He J, Starr CG, Wimley WC. A lack of synergy between membrane-permeabilizing cationic antimicrobial peptides and conventional antibiotics. Biochim Biophys Acta. 2015; 1848 (1 Pt A):8-15. doi: 10.1016/j.bbamem.2014.09.010.
[32] Brunetti J, Roscia G, Lampronti I, Gambari R, Quercini L, Falciani C, Bracci L, Pini A. Immunomodulatory and anti-inflammatory activity in vitro and in vivo of a novel antimicrobial candidate. J Biol Chem. 2016 Oct 7. pii: jbc.M116.750257.
[33] Magiorakos AP, Srinivasan A, Carey RB, Carmeli Y, Falagas ME, Giske CG, Harbarth S, Hindler JF, Kahlmeter G, Olsson-Liljequist B, Paterson DL, Rice LB, Stelling J, Struelens MJ, Vatopoulos A, Weber JT, Monnet DL. Multidrug-resistant, extensively drug-resistant and pandrug-resistant bacteria: an international expert proposal for interim standard definitions for acquired resistance. Clin Microbiol Infect. 2012; 18(3):268-81. doi: 10.1111/j.1469-0691.2011.03570.x.

## Claims

1. A combination of the antibacterial peptide KKIRVRLSA (SEQ ID NO: 1), an analogue or derivative thereof, with an antimicrobial agent selected from the group consisting of meropenem, rifampin, aztreonam and tobramycin, for use in the treatment of human or animal infections caused by one or more of the following bacterial species: *Klebsiella pneumoniae, Pseudomonas aeruginosa* and *Acinetobacter baumannii.*

2. A combination for use according to claim 1, wherein the antimicrobial agent is selected from meropenem and aztreonam and the bacterial species is *Pseudomonas aeruginosa.*

3. A combination for use according to claim 1, wherein the antimicrobial agent is selected from meropenem, aztreonam, tobramycin and rifampin and the bacterial species is *Acinetobacter baumannii.*

4. A combination for use according to claim 1, wherein the antimicrobial agent is rifampin and the bacterial species is *Klebsiella pneumoniae.*

5. A combination of the antibacterial peptide KKIRVRLSA (SEQ ID NO: 1), an analogue or derivative thereof, with an antimicrobial agent selected from the group consisting of meropenem, rifampin, aztreonam and tobramycin , for use in a method of treating infections which comprises:
a) providing a sample of biologic fluid or tissue from a subject diagnosed positive for infection by one or more of the following bacterial species: *Klebsiella pneumoniae, Pseudomonas aeruginosa* and *Acinetobacter baumannii;*
b) *testing in vitro* the responsiveness of the infectious strain to a combination as defined above
and
c) if the strain growth is inhibited *in vitro* by the combination, administering said combination to the subject.

6. A combination for use according to claims 1-5, wherein the antibacterial peptide SEQ ID NO:1 is in linear form or in dendrimeric form and particularly in two-branched or four branched-form.

7. A combination for use according to claims 1-6, wherein all the amino acids of SEQ ID NO: 1 are in either L- or D-configuration.

8. A combination for use according to claims 1-7, wherein the infection is caused by a multidrug- or extensively drug-resistant strain of said bacterial species.

9. A kit containing, in separate containers, the antibacterial peptide SEQ ID NO: 1, an analogue or derivative thereof and an antimicrobial agent selected from the group consisting of meropenem, rifampin, aztreonam and tobramycin, for use in a method of treatment of human or animal infections caused by one or more of the following bacterial species: *Klebsiella pneumoniae, Pseudomonas aeruginosa* and *Acinetobacter baumannii,* wherein the peptide and the antimicrobial agent are simultaneously or separately administered to a subject in need thereof.

10. A pharmaceutical composition comprising the antibacterial peptide SEQ ID NO: 1, an analogue or derivative thereof and an antimicrobial agent selected from the group consisting of meropenem, rifampin, aztreonam and tobramycin, together with pharmaceutically-acceptable excipients.

11. A pharmaceutical composition according to claim 10, which is in a form suitable for oral, parenteral, aerosol or topic administration.

12. A pharmaceutical composition according to claim 11, which is in the form of a tablet, capsule, injectable solution, eyewash, mouthwash, spray, aerosol, cream or ointment.

13. A kit according to claim 9 or a pharmaceutical composition according to claims 10-12, wherein the antibacterial peptide is distributed in dosage units which provide a daily amount thereof ranging from 0,5mg/kg to 50mg/Kg, preferably from 3 mg/Kg to 15 mg/Kg.
